Europäisches Patentamt

European Patent Office

Office européen des brevets

(11) Publication number: **0 266 881**
**A2**

(12) **EUROPEAN PATENT APPLICATION**

(21) Application number: **87308414.9**

(22) Date of filing: **23.09.87**

(51) Int. Cl.⁴: **G01N 21/64** , **G01N 33/53**

The title of the invention has been amended (Guidelines for Examination in the EPO, A-III, 7.3).

(30) Priority: **30.09.86 GB 8623494**
**27.01.87 GB 8701687**

(43) Date of publication of application:
**11.05.88 Bulletin 88/19**

(84) Designated Contracting States:
**DE FR GB NL SE**

(71) Applicant: **Astromed Limited**
**Innovation Centre Cambridge Science Park**
**Milton Road**
**Cambridge CB4 4GS(GB)**

(72) Inventor: **Mackay, Craig Douglas**
**53 Hamilton Road**
**Cambridge CB4 1BP(GB)**

(74) Representative: **Matthews, Heather Clare et al**
**Keith W Nash & Co Pearl Assurance House**
**90-92 Regent Street**
**Cambridge CB2 1DP(GB)**

(54) **Method and apparatus for multiple optical assaying.**

(57) A method of and apparatus for multiple assaying in which at least two components of a sample mixture are labelled by respective markers of fluorescent materials having differing peak absorption and/or emission characteristics. In one example, the sample mixture having differentially marked target components is illuminated through a scanning head (11) coupled by fibre optics (14, 16, 26, 28) to an illuminating means (18) and multiple detecting means (22, 24), filters (20, 30, 32) being incorporated in the optical system to match the response of the respective detectors to the different absorption and/or emission characteristics of the markers.

*Fig.1*

EP 0 266 881 A2

## Assay technique

### Field of the invention

This invention relates generally to assaying techniques.

### Background to the invention

A wide range of biological situations call for reliable measurements of the concentrations of specific chemical biomolecules, DNA fragments, cells and other components present in complex mixtures such as blood serum. Known techniques are able to assay for these components of the complex mixture, but in general are only able to target one component of the mixture at one time.

As an example, in immunoassays this limitation is a consequence of the assay method. Thus, in colorimetric (essentially absorbance) measurements only a single absorbance figure can realistically be measured, as most colorimetric changes are detectable over a wide spectral range. Again, in radio labelled assays it is only possible by very expensive procedures to separate decays of different energy, thereby to allow simultaneous measurements of the decay rates of different energy particles. Moreover, in luminescent assays the colour of the emitted light is set by the surrounding enzyme, so only one luminescent colour is generally available.

In practice the relevance of this problem is much wider than immunoassays. It arises with a wide variety of separation techniques such as HPLC (high pressure liquid chromatography), gel electrophoresis, paper chromatography and the like.

There are also considerable problems, even with single component assays, where the total or proportionate quantity of one component is measured. In order to achieve a useful assay, extensive use of external calibrators (controls) has to be made, and still the actual measurement depends on the details of the sample in question, such as its age, history, past processing and presentation in relation to the assay procedure used. Consequently, most assays of diagnostic value are simply of the present/absent type, with little or no attempt made to provide a meaningful quantitation of the concentration of the target chemical, antigen, antibody, cell or other target component.

### The invention

According to one aspect of the present invention, there is provided a multiple assay method wherein at least two components of a sample mixture are labelled by respective markers constituted by differing fluorescent materials of differing peak absorption and/or peak emission wavelengths, the mixture is caused to fluoresce, and the fluorescent emissions of the respective markers are optically detected and distinguished.

The method in accordance with the invention can enable essentially simultaneous assays on two or more components of a single sample. Apart from the advantage of increasing the speed and throughput of an assay system, the method thus makes it possible to establish relative concentrations of the target components, which is generally more important than absolute quantitative values. Moreover, the essentially simultaneous nature of the multiple assay means that the requirements for elaborate calibration and for provision of extensive controls are substantially reduced.

The range of fluorescent materials, eg dyes, available for use as markers means that, for almost any target component in any type of sample mixture, a suitable selection of materials can be made enabling the multiple assay to be carried out.

According to another aspect of the invention, there is provided apparatus for use in a multiple assay, comprising means for directing a beam of light on to a sample mixture having at least two target components labelled by markers in the form of differing fluorescent materials, and an optical detecting system which receives a beam of fluorescent light emitted by the sample, divides the beam into two or more parts of differing wavelength ranges respectively covering the wavelengths of the fluorescent emissions of the differing markers, and focusses the individual parts of the beam on to a multiple detecting means.

The illuminating light employed may or may not be at least in part in the visible range, depending on the selection of fluorescent materials used as markers.

According to a further aspect of the invention, there is provided a kit of reagents for use in the method of the invention, comprising supplies of at least two fluorescent materials of differing peak absorption and/or peak emission wavelengths.

## Description of drawings

The method of and apparatus for multiple assays in accordance with the invention will now be exemplified with reference to tho accompanying drawings, in which:-

Figure 1 shows a fibre optic optical illuminating and detecting system;

Figure 2 shows an optical illuminating and detecting system affording two dimensional imaging; and

Figure 3 shows a spectrographic detecting arrangement.

## Description of embodiments

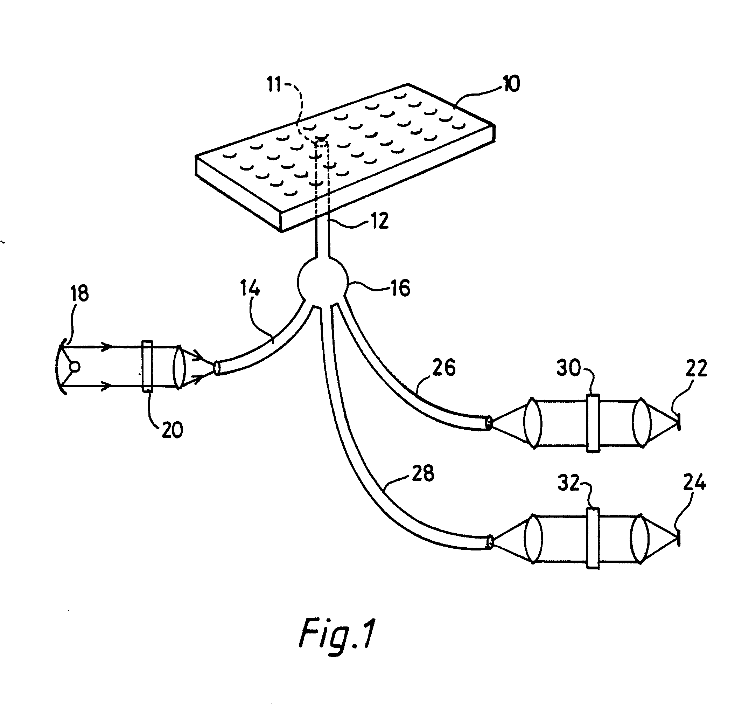

The system of Figure 1 may be practised with the Astroscan 2100 Scanner manufactured by Astromed Ltd. In this system, a Terasaki multi-well tray 10 is scanned with the head 11 of a fibre optic probe 12 brought into close proximity to the underside of each tray well in turn under the control of a computer (not shown). Tray 10 is moved in one direction by an arrangement including a drive belt and stepper motor, and the head of probe 14 is moved in the transverse direction. Each tray well contains a sample mixture having its respective target components marked by differing fluorescent dyes. The fibre probe 12 consists of several sub-bundles that are randomly brought together into the single fibre probe head 11 adjacent to the tray wells. The Astroscan 2100 Scanner uses a 3-way fibre unit 16. The stimulating light source 18, suitably filtered by filter 20 to provide the exciting wavelength, is passed down one fibre bundle 14,, and the fluorescent emission detected by single element light detectors 22, 24 located at the outputs of the other two bundles 26, 28. Appropriate emission selection filters 30, 32 are used on the output of each bundle, so that each detector is responsive to a wavelength range covering the peak fluorescent emission of one of the markers Filters 20, 30 and 32 are matched to optimise performance The Astroscan 2100 Scanner normally uses photomultiplier detectors, as it is intended for use with visible wavelength dyes. However it is possible to replace these with avalanche photodiodes (APDs) which allow longer wavelength operation (wavelength up to 1.0 microns for silicon APDs, wavelength up to 1.3 microns for germanium APDs, and wavelength up to 1.6 microns for Indium Gallium Arsenide APDs). This performance at such long wavelengths permits the use of long wavelength fluorescent dyes for a variety of applications. The outputs of the photomultipliers are digitised and sent to the controlling computer.

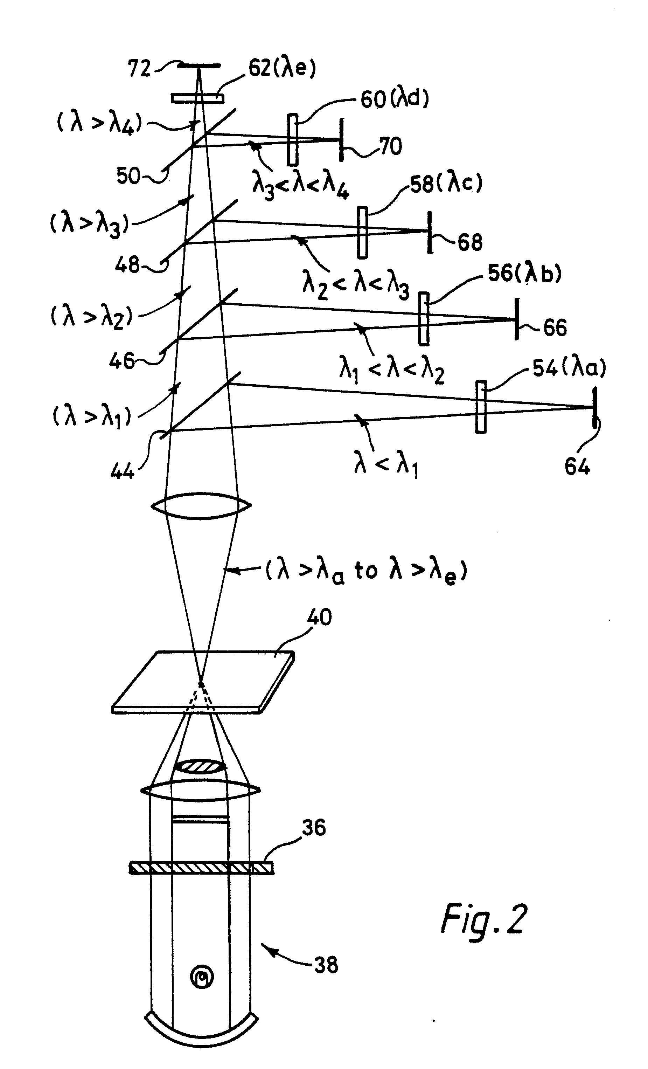

Figure 2 shows a system which uses two dimensional imaging, such as that marketed by Astromed Ltd. as the CCD 2000 Imaging System. In this system a multiply fluorescent sample 40 as described above, such as a microtiter or a Terasaki multi-well tray, or any sample of any kind capable of being imaged onto a two dimensional imaging detector, such as a microscope slide with a tissue section, smear sample or the like contacted onto it, is imaged via an optical arrangement 42 which permits the various fluorescent emission wavelengths to be separated optically by a series of dichroic filters 44 to 50 and interference filters 54 to 62. Five separate images at five different wavelengths are obtained simultaneously on five separate detector means 64 to 72. Reference 38 generally designates the collimated dark field light source, including fluorescence-stimulating filter 36. In practice, depending on the resolution needed for the assay, it is possible for beam combining optics to be used to generate five separated but adjacent images on a single detector.

Depending on the light levels available it is practicable to use a variety of detecting systems from conventional photographic or TV vidicon systems at high light levels to cooled slow scan CCD based imaging systems, such as the CCD 2000 Imaging System, for use at lower light levels.

In this connection, it is to be appreciated that although a multiple fluorescent system is advantageous, problems can arise therewith. In particular, the emission wavelengths are not single wavelengths but often have a significant wavelength range throughout which the fluorescent dye produces significant emission. The use of a cooled slow scan CCD system such as the CCD 2000 Imaging System has the advantage of high sensitivity and dynamic range, so that it is practicable to distinguish fluorescent dyes with somewhat overlapping emission spectra by the emission intensities of a sample in several adjacent wavelength regions.

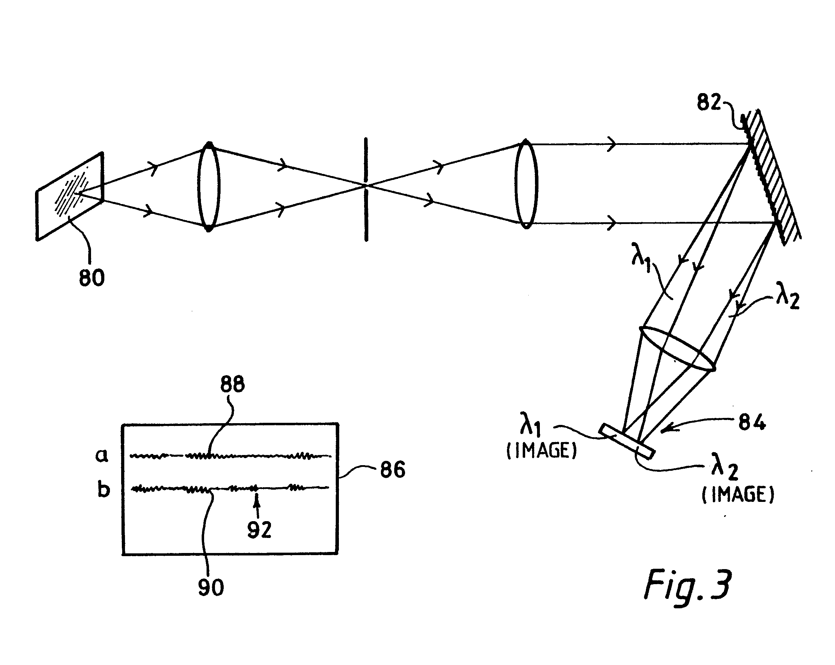

Insead of using several dichroic filters and interference filters as in the arrangement of Figure 2, it may be advantageous when dealing with complex samples containing many fluorescent target components to use a long slit spectrograph to analyse the fluorescent emission spectra of sample 80 from a spectral line imaged onto the slit of the spectrograph 82 (Figure 3). Again, the detector 84 of choice may be a cooled slow-scan CCD system such as the CCD 2000 Imaging System, but depending on the available light levels could be a more conventional TV imaging system such as a vidicon, SIT or ISIT vidicon.

The illustrated output image format 86 consists of a series of spectra of the light from each component of the sample imaged onto the spectrograph slit. Analysis of the details of the spectra allows accurate identification and quantitation of the components respectively marked by the differing fluorescent dyes mixed with the sample. Thus, each element of the spectrograph slit produces a spectral image line 88 or 90 for a given target component. Due to the fluorescent marker associated with the marked component imaged at 90, the spectral line contains an additional emission peak 92 when compared with the spectral line 88.

The above described apparatus may be employed to practice the method of the invention in various fields of application. For example, in cell death studies, chemicals (such as Carboxy Fluorescein diacetate C-FDA) may be used to stain live cells while Propidium Iodide (PI) may be used to stain DNA from dead cells. In any cellular experiment the ratios of live to dead cells in a sample may be obtained by measuring the ratios of C-FDA to PI emission after appropriate calibrations. Such tests arise when looking at monoclonal reagent tests, tests of herbicides on plant cells, and drug tests. These tests may, for example, be conducted with a system of the type of the Astroscan 2100 Scanner, or a two colour version of the Imaging System.

Again in determining cell type ratios, differing markers may be attached to different monoclonals specific to different cell types, whereby it is practicable to measure directly the ratios of different kinds of cells in a sample. For example there exist diseases where it is important to know the relative proportions of the different kinds of T and B cells in a patient's blood, and how these ratios are responding to treatment. Using a differing fluorescent marker for each kind of cell relevant to that particular test allows these ratios to be established with considerable accuracy, using a system such as that described in relation to Figure 2.

With respect to antibody ratios, in any patient there are background antibody levels that arise from earlier infections such as the carrier childhood diseases. By conducting a multi-fluorescent assay for patient antibodies in relation to a current illness, in parallel with a childhood disease antibody test or other standard for comparison, the progress of a disease can be followed with considerable accuracy. Such a procedure is valuable in plotting the advance of a disease such as AIDS and gives rapid indication whether the development of the disease in a patient has been halted.

The multiple fluorescence assay can also be used to create a pecise, rapid assay for viruses such as HTLV-I, HTLV-III, Measles etc. The procedure would be as follows. The appropriate target T cells would be extracted from a whole blood sample using, for example, the magnetic beads sold under the trade name Dynabeads by Dynal A.S., Oslo, coated with the appropriate T cell specific antibody. An antibody to the particular virus would then be introduced so as to lyse the virus infected cells. As before live and dead cells would be differently stained with two colours of fluorochrome and the ratios of live to dead cells determined by the double fluorescence technique would give a direct assessment of the state of development of the viral infection in question.

Drug impurity assays are enabled by the use of fluorescent markers on monoclonal antibodies in a pharmaceutical product, allowing an accurate measurement of their relative concentrations at different stages of the manufacturing process. Use of one marker as a constant control (applied to a harmless impurity added for the purpose) allows a much easier and cheaper approach to pharmaceutical quality control and thus a lower risk of dangerous side effects in pharmaceutical products.

Yet again, in low temperature fluorescence, a surprisingly large number of proteins and other more complex molecules spontaneously fluoresce at low temperatures and the wavelength and time course of the fluorescence is an important indicator of the particular protein causing the fluorescence. The use of low-temperature protein fluorescence is potentially very valuable since it allows the protein to be assayed without any external chemical reaction having to occur. Accordingly the protein, once identified, may be isolated for further analysis. As an analytic tool the spectroscopic system of Figure 3 is most flexible, since it allows the most subtle distinctions of fluorescence spectra to be made.

Various modifications of the above described embodiments are possible within the scope of the invention hereinbefore defined.

**Claims**

1. Apparatus for use in a multiple assay, comprising means for directing a beam of light on to a sample mixture having at least two target components labelled by markers in the form of differing fluorescent materials, and an optical detecting system which receives a beam of fluorescent light emitted by the sample, divides the beam into two or more parts of differing wavelength ranges respectively covering the wavelengths of the fluorescent emissions of the differing markers, and focusses the individual parts of the beam on to a multiple detecting means.

2. Apparatus according to claim 1, including a source for producing illuminating light over a wavelength range covering the wavelengths which cause the markers to fluoresce.

3. Apparatus according to claim 1 or claim 2, wherein a plurality of sample mixtures each having marked target components are carried by a multi-well tray and the tray wells are scanned by relative movement in two coordinate directions between the tray and an optical scanner coupled to the illuminating means and a plurality of detecting means.

4. Apparatus according to claim 3, wherein the illuminating means and the detecting means are coupled to a scanning head through fibre optic bundles.

5. Apparatus according to claim 3 or claim 4, wherein the coupling means for each of the plurality of detecting means includes a selection filter for matching the response of a given detecting means to the peak fluorescent emission of one of the markers.

6. Apparatus according to any of claims 1 to 5, wherein the detecting means comprise a plurality of photomultipliers and means are provided for digitising the outputs thereof for computerised processing.

7. Apparatus according to any of claims 1 to 5, wherein the detecting means is a slow scan CCD based imaging system.

8. A method of multiple assaying wherein at least two components of a sample mixture are labelled by respective markers constituted by differing fluorescent materials of differing peak absorption and/or peak emission wavelengths, the mixture is caused to fluoresce, and the fluorescent emissions of the respective markers are optically detected and distinguished.

9. A method according to claim 8, wherein the system is optimised to match the means for optical detection with respect to the absorption and/or emission characteristic spectrum of the markers.

10. A method according to claim 8 or 9, when applied to cell death studies by differentially marking live and dead cells in the sample mixture, or when applied to determination of cell type ratios by differentially marking monoclonals specific to the different cell types, or when applied to doing impurity assays by differentially marking monoclonal antibodies in the product, or when applied to low temperature fluorescence of proteins and analogous complex molecules.

*Fig.1*

*Fig.2*

Fig.3